# EUROPEAN PATENT APPLICATION

(11) **EP 3 836 155 A1**
(43) Date of publication of application: **16.06.2021**
(21) Application number: 19215527.3
(22) Date of filing: 12.12.2019
(51) Int. Cl.: G16H 30/20, G16H 30/40

(54) **SCREEN CAPTURING VIA MOBILE COMPUTING DEVICES USING MATRIX CODE**

(71) Applicant: Koninklijke Philips N.V., 5656 AG Eindhoven (NL)
(72) Inventor: SAALBACH, Axel, 5656 AE Eindhoven (NL); SCHULZ, Heinrich, 5656 AE Eindhoven (NL); GRASS, Michael, 5656 AE Eindhoven (NL); BALTRUSCHAT, Ivo Matteo, 5656 AE Eindhoven (NL)
(74) Representative: Philips Intellectual Property & Standards

(57) **Abstract**

The present invention relates to a device for mobile device-based image analysis, comprising: an image processor unit; a display unit; and an image information source unit; wherein the image information source device is configured to provide an image; wherein the image processor unit is configured to generate at least one matrix code based on the provided image; wherein the display unit is configured to display the provided image.

## Description

### FIELD OF THE INVENTION

The present invention relates to systems and methods for image analysis via mobile computing devices using screen capturing, as well as to a computer program element and a computer readable medium.

### BACKGROUND OF THE INVENTION

In recent years, the use of artificial intelligence for image analysis showed promising results in a broad range of applications from computer vision tasks to medical image analysis. With the constantly improving hardware of mobile computing devices (i.e. camera resolution, compute power), and the increasing availability of high-speed internet connections, it has become possible to employ (always up-to-date) AI applications in the daily routine.

At the same time, medical devices such as X-Ray systems or diagnostic applications are often employed in a secure environment, thus, without internet access, and less frequent update cycles, due to quality and regulatory aspects.

Therefore, the use of mobile computing devices - such as smart phones, tablets or any equivalent - provides an interesting alternative to bring AI technology into the clinical environment.

### SUMMARY OF THE INVENTION

It would be advantageous to have an improved technique for screen capturing in the context of image analysis via mobile computing devices.

The object of the present invention is solved with the subject matter of the independent claims, wherein further embodiments are incorporated in the dependent claims. It should be noted that the following described aspects and examples of the invention apply also to the device, the method, as well as to the computer program element and a computer readable medium.

In a first aspect, there is provided a device for mobile device-based image analysis. The device comprises: an image processor unit; a display unit, and an image information source unit. The image information source device is configured to provide an image; wherein the image processor unit is configured to generate at least one matrix code based on the provided image; wherein the display unit is configured to display the generated matrix code and the provided image.

A matrix code is a two-dimensional barcode, a.k.a. matrix barcode or 2D code. A well-known example of a matrix code is a matrix code. It can be used to reliably represent, or encode, digital data, possibly using redundancy.

In an embodiment this displaying of the matrix code might be performed by means of an overlay in the display and/or by means of an alternative display mode, rather than to change an image itself.

In other words, the present invention for example provides solutions and systems for
i) Generating and displaying the information;
ii) Using the information (e.g. on a mobile device).

In this manner, for example, the device is configured to add a matrix code to a medical image or to replace the displayed image with a matrix code. The matrix code can include one or more of the following data:
(i) Patient metadata; and/or
(ii) A relevant part of the image. This avoids image quality loss of the relevant part of the image in the transmission from display to device, but limits the data quantity to a level that can still be communicated via a matrix code; and/or
(ii) Extracted features from the image processing domain (SIFT, SURF, HOG, or the like), as well as features derived from Deep Learning networks such as ResNets or DenseNets.

In an exemplary embodiment of the present invention, for example, the device is configured to subsequently show the matrix code after the respective image was displayed on a display.

In other words, for example, an alternative display mode for medical images and meta data) is provided by the present invention, which allows for an easier and more robust capturing of image information via mobile devices. In particular, this includes the display of specific information such as, image data, derived image features as well as meta information as a (matrix) bar code.

In an exemplary embodiment of the present invention, for example, the image processor unit is configured to add the at least one matrix code, which includes raw image data of at least a part of the image.

In an exemplary embodiment of the present invention, for example, the image processor unit is configured to add the at least one matrix code, which includes extracted image features of at least a part of the image.

In an exemplary embodiment of the present invention, for example, thimage processor unit is configured to generate the extracted image features of at least a part of the image by deep-learning networks.

In an exemplary embodiment of the present invention, for example, the image processor unit is configured to generate the extracted image features of at least a part of the image by using a convolutional neural network.

In an exemplary embodiment of the present invention, for example, the image information source unit is configured to be connected to a computed tomography system or a magnetic resonance imaging system or an X-ray imaging system or a medical imaging system or a diagnostic imaging system.

In an exemplary embodiment of the present invention, for example, the image processor unit is configured to encrypt any data as provided by the added at least one matrix code.

In an exemplary embodiment of the present invention, for example, the image processor unit is configured to add the at least one matrix code, which includes metadata of a patient.

In a second aspect, there is provided a mobile device configured to communicate with the device according to the first aspect or any implementation of the first aspect, wherein the mobile device is configured to perform image analysis on the displayed and provided image, wherein optionally the mobile device is configured to perform the image analysis using artificial intelligence algorithms.

In a third aspect, there is provided a method for mobile device-based image analysis, comprising the following steps of:
As a first step, providing an image by means of an image information source unit;
As a second step, generating at least one matrix code based on the provided image by means of an image processor unit; and
As a third step, displaying the provided image by means of a display unit.

In an exemplary embodiment of the present invention, for example, the method further includes the step of adding the at least one matrix code to the displayed image and displaying the provided image with the added at least one matrix code.

The adding at least one matrix code to the provided image by means of an image processor unit is a viable option.

According to an exemplary embodiment of the present invention, only the image is displayed and the added at least one matrix code may be not displayed, but transmitted via wireless data transmission to the mobile computing device.

According to another aspect, there is provided a computer program element controlling one or more of the apparatuses as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a device for supporting image analysis using mobile devices according to an exemplary embodiment of the present invention;
Fig. 2 shows a method for mobile device-based image analysis according to an exemplary embodiment of the present invention;
Fig. 3 shows an example of a display showing an image and matrix code used to support the smart phone-based image analysis according to an exemplary embodiment of the present invention; and
Fig. 4 shows an example of mobile phone or tablet systems for image analysis via mobile computing devices using screen capturing.

### DETAILED DESCRIPTION OF EMBODIMENTS

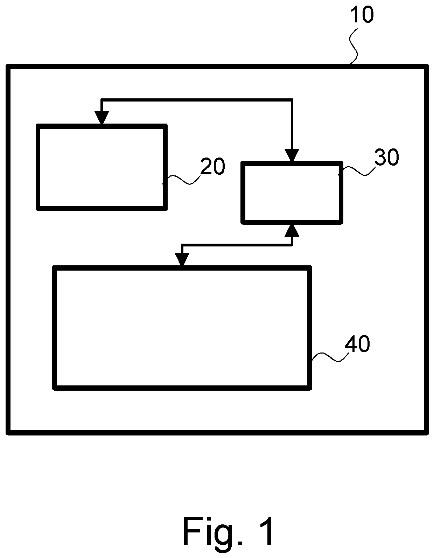

Fig. 1 shows a schematic set up of an example of a device for image analysis based on mobile devices according to an exemplary embodiment of the present invention. The device 10 for mobile device-based image analysis comprises an image processor unit 20, a display unit 30, and an image information source unit 40.

The image information source device 40 is configured to provide an image in terms of any medical image of a diagnostic system, for example a CT or an MR imaging system or an X-ray based imaging system.

Mobile devices can be used to bring artificial intelligence, AI, technology for image analysis into the clinical environment. Image capturing can be done by the mobile device camera taking a picture from a monitor or by other upload means.

Direct upload requires a compatible and interconnected system, which is not always available. The direct capture of images from a display as shown above also has certain disadvantages: i) loss of quality image, ii) invasion of privacy of patient.

To avoid these disadvantages, in an embodiment of the invention the device displays a matrix code in addition to the image to support a mobile device to perform image analysis. This adding of the matrix code to the image may be performed by an overlay, a transparent merging of the matrix code and the image. In an alternative embodiment, the matrix code is displayed subsequently to the image, e.g. the image and the QR are displayed alternately, in an interval interlacing.

According to an exemplary embodiment of the present invention, the matrix code for example embeds patient metadata, relevant medical image data and/or derived image features.

According to an exemplary embodiment of the present invention, the matrix code is used on images displayed on X-ray systems or diagnostic applications and can be read using mobile devices for analyzing images.

The image processor unit 20 is configured to generate at least one matrix code based on the provided image. According to an exemplary embodiment, the image processor unit 20 is configured to add at least one matrix code to the provided image, for example in an interlacing mode or by merging in transparent overlaying techniques the matrix code and the image.

The display unit 30 is configured to display the provided image, optionally with the added at least one matrix code.

According to an exemplary embodiment of the present invention, the use of such codes is performed in order to make medical information available to mobile device users. In this context, different scenarios can be considered:
According to an exemplary embodiment of the present invention, displaying of image information is performed:
In this context, one or more images or a part of an image (e.g. a ROI covering a lesion) are selected by the user, and are encoded and displayed as a matrix code. With the limitations of recent matrix codes, for example 2953 bytes, this is suitable only for sufficient small image patches, or to scenarios where the image can be distributed across multiple matrix codes.

Further, it is noted that most image analysis algorithms operate on a substantially reduced image resolution or on only a fraction of the image. This means that for example only a fraction of the image need to be encoded, for example typically 224 x 224 pixels. Compared to a conventional screen shot, the encoding of the image part ensures the correctness of the transmission of the relevant part (whereas a screen shot of the image part would not be an accurate reproduction).

According to an exemplary embodiment of the present invention, displaying of feature information is performed: Many image processing applications operate on derived image features rather than the original image intensities. Next to standard features, like for instance SIFT, SURF, HOG or equivalent methods, Deep Learning networks (like ResNets, DenseNets), have proven to be powerful feature extractors.

According to an exemplary embodiment of the present invention, a ResNet-34 allows for the representation of a chest X-ray in terms of 1024 features, which can be directly encoded in a matrix code while the derived features allow for a classification of the image. According to an exemplary embodiment of the present invention, displaying of meta data is performed: In other applications, already a reliable access to the patient meta data would be beneficial (e.g. in order to support annotations / labeling of medical data using mobile devices).

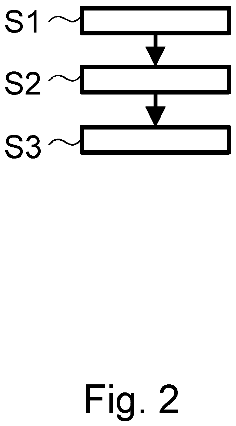

Fig. 2 shows a method for mobile device-based image analysis according to an exemplary embodiment of the present invention. The method comprises:
As a first step of the method, providing S1 an image by means of an image information source unit 40 is performed.

As a second step of the method, generating S2 at least one matrix code to the provided image by means of an image processor unit 20 is performed.

As a third step of the method, displaying S3 the provided image, optionally together with the added at least one matrix code, by means of a display unit 30 is performed. According to an exemplary embodiment of the present invention, the method further includes the steps of generating at least one matrix code from the image and displaying the at least one matrix code.

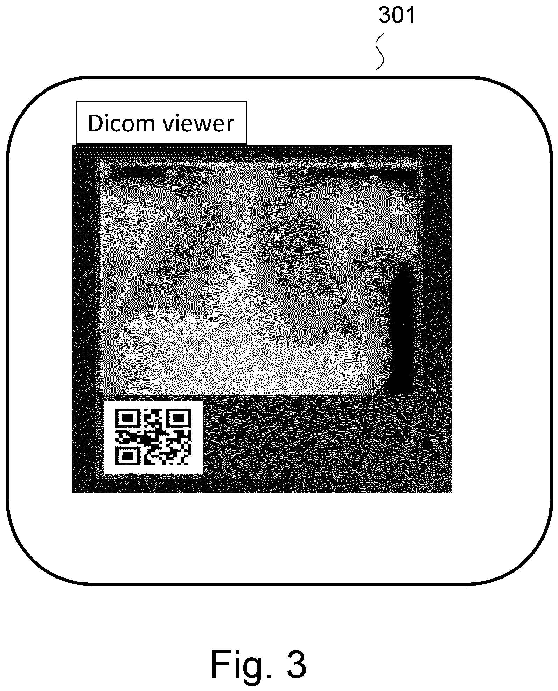

Fig. 3 shows an example of an image on a display 301 and an illustration of a matrix code on the same display to be used for the smart phone-based image analysis (after capturing by the smart phone) according to an exemplary embodiment of the present invention.

The matrix code may encode patient data, for example such as the patient ID, name, or the like or image features in order to facilitate task such as image labeling, and/or may have encoded therein a part of the image and/or certain image features.

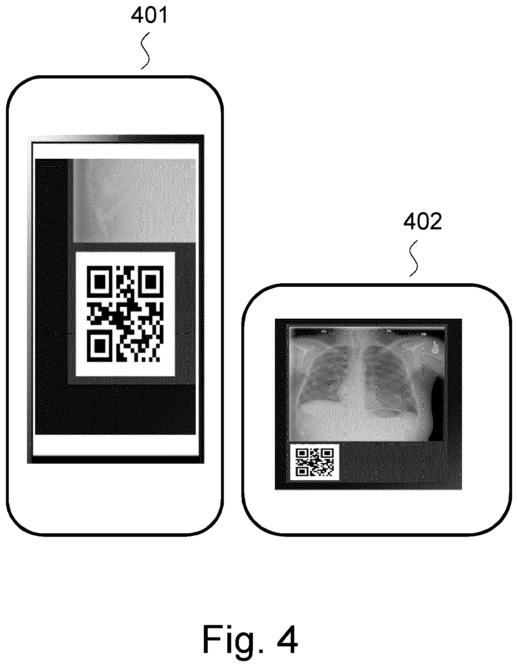

Fig. 4 shows on the left side an example of a mobile phone 401 capturing or having captured, from the display of Fig. 3, the matrix code and possibly also at least part of the image via a camera. The image shown on the mobile phone screen has been captured using a mobile phone camera from the display images and matrix code as visible in Fig. 3. This image can for example be used for classification purposes using a dedicated ResNet model for Chest-X ray analysis.

In another exemplary embodiment, the mobile phone is configured to perform image analysis on the displayed and provided image, wherein optionally the mobile phone or mobile device is configured to perform the image analysis using artificial intelligence algorithms.

Fig. 4 shows on the right side an example of a tablet 402 for image analysis via mobile computing devices using screen capturing similar to the example of a smart phone described above.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above.

Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both a computer program that right from the beginning uses the invention and computer program that by means of an update turns an existing program into a program that uses the invention.

Further, according to a further exemplary embodiment of the present invention, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for supporting mobile device-based image analysis, comprising:
- an image processor unit (20);
- a display unit (30, 301); and
- an image information source unit (40);
wherein the image information source device (40) is configured to provide an image; wherein the image processor unit (20) is configured to generate at least one matrix code based on the provided image; wherein the display unit (30, 301) is configured to display the provided image and to display the at least one matrix code.

2. The device according to claim 1, wherein the at least one matrix code is generated from the image contents.

3. The device according to any one of the claims 1 to 2, wherein the at least one matrix code encodes raw image data of at least a part of the image.

4. The device according to claim 2 or 3, wherein the at least one matrix code encodes extracted image features of at least a part of the image.

5. The device according to claim 4, wherein the image processor unit (20) is configured to generate the extracted image features of at least a part of the image by deep-learning networks.

6. The device according to any one of the claims 4 to 5, wherein the image processor unit (20) is configured to generate the extracted image features of at least a part of the image by using a convolutional neural network.

7. The device according to any one of the claims 1 to 6, wherein the image information source unit (40) is configured to be connected to a computed tomography system or a magnetic resonance imaging system or an X-ray imaging system or a medical imaging system or a diagnostic imaging system.

8. The device according to any one of the claims 1 to 7, wherein the image processor unit (20) is configured to encrypt any data in the generated at least one matrix code.

9. A mobile device (401) configured to capture a matrix code from the device according to any one of the claims 1 to 8, wherein the mobile device is configured to decode the matrix code and to perform image analysis on the image information contained in the matrix code, wherein optionally the mobile device is configured to perform the image analysis using artificial intelligence algorithms.

10. A method (100) for mobile device-based image analysis, the method comprising the following steps of:
- providing (S1) an image by means of an image information source unit (40);
- generating (S2) at least one matrix code based on the provided image by means of an image processor unit (20); and
- displaying (S3) the provided image by means of a display unit (30) and the at least one matrix code.

11. The method according to claim 10, wherein the method further includes the steps of generating at least one matrix code from the image.

12. The method according to claim 10 or 11, wherein the at least one matrix code includes raw image data of at least a part of the image.

13. A method for mobile device-based image analysis, the method comprising the following steps of:
- capturing a matrix code based on an image;
- decoding information on the image from the matrix code;
- performing image analysis of the decoded information, wherein optionally the mobile device is configured to perform the image analysis using artificial intelligence algorithms;
- showing the results of the performed image analysis.

14. A computer program element for controlling a device according to any one of the claims 1 to 9, which when executed by a processor is configured to carry out the method of claim according to any one of the claims 10 to 13.

15. A computer-readable storage medium comprising instructions which, when executed by a computer, cause the computer to carry out the method according to any one of the claims 10 to 13.
